# EUROPEAN PATENT APPLICATION

(11) **EP 0 590 918 A1**
(43) Date of publication of application: **06.04.1994**
(21) Application number: 93307653.1
(22) Date of filing: 28.09.1993
(51) Int. Cl.: C12M 1/34

(54) **Method of critical specimen resistance testing**

(30) Priority: 30.09.1992 US 954836
(71) Applicant: Becton Dickinson and Company, Franklin Lakes, New Jersey 07417-1880 (US)
(72) Inventor: Gabriele, Thomas L., Timonium, Maryland 21039 (US)
(74) Representative: Ruffles, Graham Keith

(57) **Abstract**

A method of critical specimen resistance testing that tests the resistance of an infecting organism to a prescribed drug. After a technician or physician has identified the presence of a microorganism and prescribed a particular antimicrobic to inhibit the growth of that microorganism, a fluid specimen taken from the patient is injected into a first sterile vial containing a growth medium and a resin medium and a second sterile vial containing only the growth medium. The second vial is also injected with the prescribed antimicrobic and both vials are then agitated and incubated to promote the growth of the microorganism. Based upon a comparison between the growth in both vials, the effect of the prescribed antimicrobic on the microorganism is determined. Moreover, by periodically monitoring the growth of the microorganism in a treated vial and an untreated vial and generating growth response curves and then comparing those curves to growth response curves of known microorganisms being treated by known antimicrobics, the genus and species of the microorganism may also be identified.

## Description

### BACKGROUND OF THE INVENTION

### 1. Field of the Invention

The present invention relates to a method of critical specimen resistance testing and, more particularly, relates to a method of blood culture testing that provides early information regarding the resistance of microorganisms to prescriptions, drugs or antimicrobics.

### 2. Background Description

As practitioners-in-the-art of clinical microbiology testing are aware, it has long been a goal to facilitate economical therapy more efficiently for patients to decrease the length of their stay in a hospital. Antimicrobic resistance testing is ever increasing in importance since it addresses the immediately pertinent diagnostic question of whether an infecting organism is resistant to a drug or antibiotic prescribed by a physician.

Based upon an examination of a patient and the preparation of an initial diagnosis, the physician may prescribe a particular drug or antibiotic to inhibit growth of a particular bacteria. It is important for the physician to select the best antibiotic for the patient. However, for a physician to select the best antibiotic it may be necessary for the bacteria in a fluid specimen, such as blood, to be isolated in a laboratory and tested using numerous antibiotics with the results of these tests not being available for 48 hours. Therefore, there is the need for a method of determining whether the prescribed antibiotic is inhibiting the growth of the bacteria as soon as possible and, preferably, within 48 hours.

Presently, there is a variety of tests available for selecting the best antibiotic to kill the bacteria in a specimen but, as noted above, these tests take 48 hours. A number of these tests are described in U.S. Patent No. 4,448,534, i.e., Minimum Inhibitory Concentration test (MIC) and Antimicrobial Susceptibility Test (AST).

Susceptibility tests are laboratory procedures that determine to what degree bacteria are inhibited by selected antimicrobial agents, however, these tests cannot be performed until a blood specimen has been cultured and bacterial colonies in the specimen have been isolated. The outcome of these susceptibility tests may yield qualitative results or quantitative results. The qualitative results indicate whether the bacteria is resistant or susceptible to a particular antibiotic and the quantitative results indicate how susceptible the bacteria is to various concentrations of an antibiotic.

The most frequently used susceptibility tests are the disc diffusion test, which is a qualitative test, and the dilution test, which is a quantitative test. The disc diffusion test, commonly known as the Kirby-Bauer method, is performed by inoculating the surface of a Mueller-Hinton agar plate with a standardized suspension of bacteria and then applying filter paper discs, each disc being impregnated with a single concentration of a different antimicrobial agent or antibiotic. The plate is then incubated for 16 to 18 hours to allow for bacterial growth and the zones of inhibition around each disc are measured to determine the susceptibility of the bacteria to each antibiotic.

The dilution tests, on the other hand, are quantitative tests that permit a technician or physician to accurately determine the concentration required to treat a specific infection, the lowest concentration being called the Minimum Inhibitory Concentration (MIC). The most common methods of dilution used to determine the MIC are Agar dilution and Broth dilution.

The Agar dilution method involves preparing agar plates with different concentrations of the antibiotic, spot inoculating the plates with a standardized suspension of the bacteria and then incubating the plates overnight. The Broth dilution method involves placing different concentrations of the antibiotic in wells or tubes containing a broth, adding a standardized suspension of bacteria to each well or tube and then incubating the mixtures for 16 to 20 hours. In both methods the plates, tubes or wells are then examined for bacterial growth, and the MIC is identified by finding the receptacle having the lowest concentration of antibiotic with no bacterial growth.

An extension of the MIC test is the MIC/ID test, which not only provides qualitative and quantitative results, but also identifies the genus and species of the bacteria. However, when using MIC or MIC/ID the bacteria must be isolated and grown in concentration by incubation, which takes 48 hours. Therefore, MIC/ID also does not address the time delay problem of the other testing methods.

In addition, other qualitative and quantitative susceptibility tests are described in U.S. Patent No. 4,448,534.

Unfortunately, each of the above-described tests take 48 hours to perform and generate results and, therefore, do not provide information concerning the resistance of the bacteria to the specific antibiotic prescribed as early as is necessary. In fact, after 48 hours, the tests may only be confirmatory, since information received at that time would merely compliment the drug's efficacy evidenced by the patient's response to therapy during those first 48 hours.

Since it is highly undesirable for the only source of information concerning bacteria's resistance to a drug to be the improvement or deterioration of a patient's general health, there is the need for a test that determines if the bacteria are being inhibited by the drug as soon as possible.

In addition, as those skilled in the art are aware, each of the above-described tests has the potential of generating inaccurate results, which can have serious implications for the patient. Therefore, it would also be valuable to have a back-up or second level test performed in parallel to those described-above so to reduce the likelihood that a major error is overlooked.

### SUMMARY OF THE INVENTION

The present invention overcomes the problems identified in the background material by providing a method for critical specimen resistance testing that permits a bacteria's resistance to a prescribed antimicrobic or antibiotic to be identified early, possibly within several hours. In addition, the present invention aids in determining whether any other susceptibility tests are necessary and complements the results of other susceptibility tests so to reduce the liklihood that a major error will go unnoticed.

A preferred embodiment of the method includes injecting a small quantity of blood from a patient through a sealing rubber septum into a first sterile vial containing a growth medium and a resin medium and injecting another small quantity of the patient's blood into a second sterile vial containing only the growth medium. The second vial is then injected with a prescribed antimicrobic. Both of the vials are then agitated, incubated and monitored for bacterial growth. Based upon a comparison between the growth in both vials and the first sign of growth in the second vial, it is determined whether the prescribed therapy should be enhanced or modified. However, if no or significantly retarded growth is seen in the second vial, the initially prescribed antimicrobic is maintained and no further change in the prescription is necessary.

Alternatively, a first sterile vial containing a resin medium is injected with a quantity of blood and then incubated to allow any bacteria in the blood to grow. If bacterial growth is detected, about half the culture in the first vial is placed in a second sterile vial to grow naturally and the other half of the culture is placed in a third sterile vial containing a prescribed antimicrobic. Then, after a period of time, the difference in bacterial growth in the two vials indicates whether the prescribed antimicrobic is inhibiting the growth of the bacteria.

Moreover, by periodically monitoring the growth of the bacteria in a treated vial and an untreated vial to generate growth response curves and then comparing those curves to growth response curves of known bacteria being treated by the prescribed drug, the genus and species of the bacteria may be identified.

Thus the present invention provides a faster and more effective method of testing the resistance of bacteria in a blood sample to a prescribed drug than presently available or known in the art. The present invention provides quick results, which can indicate possible resistance to a drug as early as the "afternoon rounds" of the first day of a patient's hospital stay. Since the present invention provides this information early it provides for effective therapy quicker, accelerates patient recovery and reduces the chance of a protracted illness being caused by resistance of bacteria to a prescribed drug.

These and other aspects, features and advantages of the present invention will become apparent from the following detailed description taken in conjunction with the accompanying drawings.

### DESCRIPTION OF THE DRAWINGS

Fig. 1 is a perspective view of an apparatus capable of practicing the present invention;
Fig. 2 is a side view of a vial used in practicing the present invention;
Fig. 3 is a block diagram of a preferred control system for the apparatus shown in Fig. 1;
Fig. 4 is a flow chart of a preferred method of the present invention;
Fig. 5 is a flow chart of an alternative method of the present invention; and
Fig. 6 is a plot showing growth response curves for a treated vial and an untreated vial when using the present invention.

### DETAILED DESCRIPTION

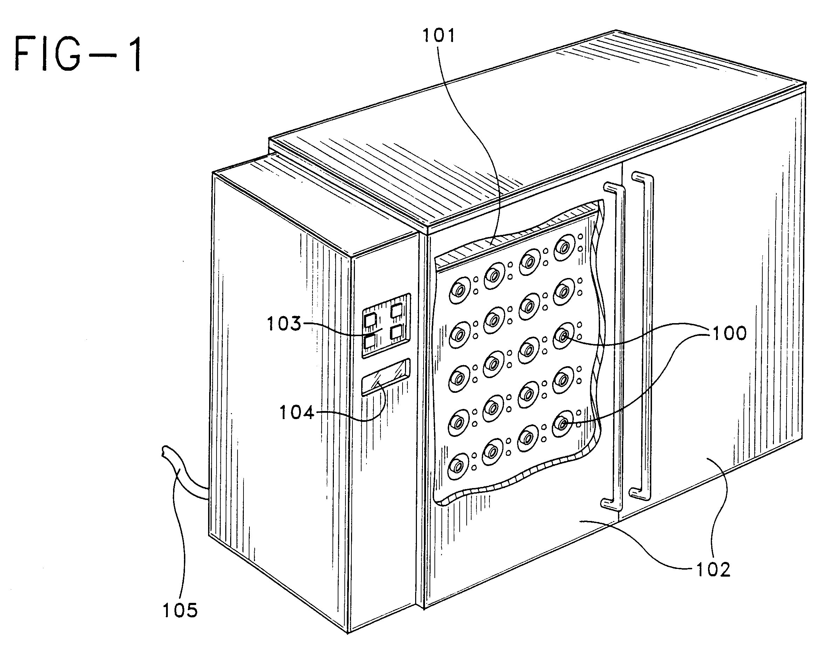

An example of a preferred apparatus for performing a preferred method embodying the principles and concepts of the present invention is shown in Fig. 1, wherein vials 100 to be tested for biological activity are held and arranged in an X-Y matrix 101 within the apparatus. In a preferred embodiment of the apparatus, X-Y matrix 101 contains up to 240 vials and is movably mounted in the apparatus for vibrational movement so to agitate the contents of each of the vials during testing.

Vials 100 are enclosed in the apparatus and protected from external environment when under test by a pair of hinged doors 102 on the front of the apparatus. Heating means (not shown) are also provided in the apparatus for incubating the vials in X-Y matrix 101 at a temperature conducive to metabolism of microorganisms, e.g., 37°C, when doors 102 are in a closed position. Therefore, the apparatus provides for the simultaneous agitation and incubation of all of the vials so to provide an ideal environment for the growth of bacteria within each vial. An example of such an apparatus is the BACTEC 9240®, which is a non-invasive blood culturing system that is sold by Becton, Dickinson and Company.

In addition, a display 103 is provided on the front of the apparatus in Fig. 1 for indicating the operational status of the apparatus, and a control panel 104 provides a plurality of switches, i.e., for testing the apparatus or turning the apparatus on and off. Cable 105 connects the apparatus to a control system 300, discussed below, which controls the overall operation of the apparatus.

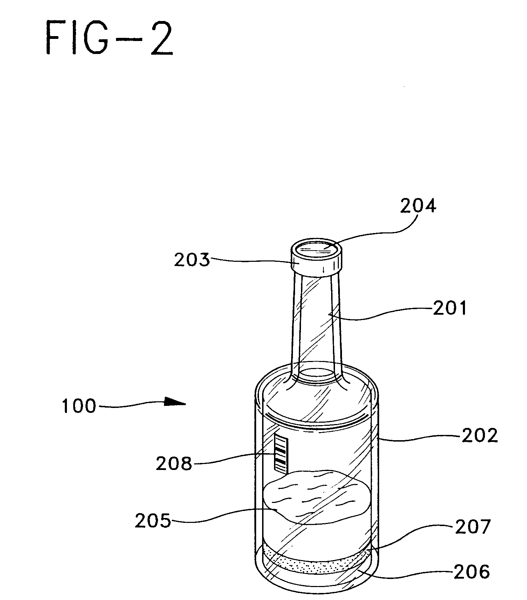

A side view of a preferred vial 100 for use with the present invention is shown in Fig. 2. Vial 100 includes a neck portion 201 and a base portion 202, with neck portion 201 having a smaller diameter than base portion 202. A cap 203 seals the open upper end of neck portion 201 and includes a rubber septum 204 that permits a needle to be inserted into vial 100 for injecting a fluid specimen into vial 100 and then reseals the open end of vial 100 when the needle is withdrawn. Vial 100 is shown as including a growth medium 205, which stimulates the growth of bacteria that may be in the fluid that is injected into the vial when the vial is incubated and agitated.

In the embodiment being described, a fluorescent-based CO₂ sensor 206 is mounted at the bottom of base portion 202 for non-invasively detecting the presence of CO₂ in vial 100. As bacteria in the fluid specimen injected through septum 204 into vial 100 grows in growth medium 205, bacteria metabolism generates CO₂. Therefore, the detection of CO₂ in vial 100 by sensor 206 indicates that bacteria are growing within the vial. In addition, vial 100 in Fig. 2 contains an optional resin medium 207 to absorb any antibiotics or drugs that may be in the vial. An example of such a vial is the vial sold by Becton, Dickinson and Company for use in the BACTEC 9240®.

In addition, it is preferable for each vial 100 to contain a separate and distinct bar code label 208 to provide efficient tracking for each vial and minimize reporting errors.

If the fluid specimen that is injected into each vial 100 is blood, the apparatus provides a non-invasive blood culturing system that periodically and concurrently monitors, agitates and incubates the vials. Since each vial 100 contains a fluorescent-based CO₂ sensor 206 that continuously monitors the blood culture in the vial, the blood culturing system based upon the above-described apparatus provides the earliest possible detection of bacterial growth in each vial 100. In addition, the system provides a continuous source of periodic data concerning the growth of bacteria in the blood culture in each vial 100 which can be stored and analyzed at a subsequent time.

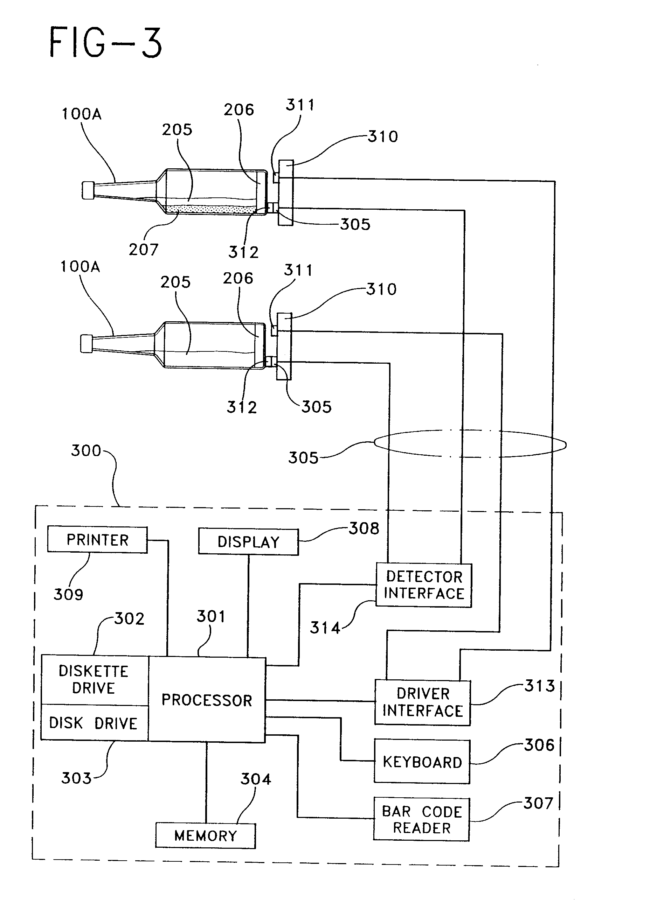

Fig. 3 shows a block diagram of an exemplary control system 300 for the apparatus shown in Fig. 1. Control system 300 includes a processor 301 that is connected to a diskette drive 302 and a disk drive 303 wherein both drives 302 and 303 are used to store and retrieve programs and data used to operate and control the apparatus. Both drives 302 and 303 are conventional drives, with diskette drive 302 having a removable storage medium and disk drive 303 having a non-removable storage medium with a larger storage capacity than the removable storage medium in diskette drive 302. Control system 300 also includes a memory 304 that temporarily stores programs that are currently being executed by processor 301 and data being received from drives 302 and 303 or other peripheral devices, i.e., detectors 305.

Processor 301 controls the apparatus in Fig. 1 through cable 105 in accordance with program instructions stored in memory 304. An operator selects the programs to be executed by processor 301 using a keyboard 306 or a bar code reader 307, and the results of tests being performed by the apparatus and overall system status information are displayed on a display 308 or printed at printer 309. Bar code reader 307 is also used to read bar code 208 on each vial 100 and provide that information to processor 301 for tracking each vial 100.

As shown in Fig. 3, a test assembly 310 is associated with each vial 100A and 100B for testing each vial for the presence of CO₂ gas. Preferably, each test assembly 310 includes a light source 311, a filter 312, and a photodiode detector 305 and is controlled by processor 301 over cable 105 through a driver interface 313 and a detector interface 314.

In operation, as light is generated by light source 311 and directed into fluorescent-based CO₂ sensor 206 at the base of each vial 100A and 100B, fluorescent-based sensor 206 emits differing quantities of light depending upon the amount of CO₂ detected by sensor 206. For example, the more CO₂ in the vial, the more fluorescent light in emitted by sensor 206. The emitted light passes through filter 312 and is received by detector 305, which then transmits data concerning the level of light being received to detector interface 314.

Processor 301 drives each light source 311 through driver interface 313 and then samples the data being received by detectors 305 at detector interface 314. Based upon the information received from detector interface 314, in response to the light being generated by a corresponding light source 311, processor 301 determines the relative amount of CO₂ in each vial 100A and 100B and stores the results of these tests in memory 304 or on drives 302 and 303. In addition, processor 301 selects which test assembly 310 is to be used and, therefore, can perform tests using the test assemblies 310 either sequentially or in parallel.

It is to be understood that control system 300, shown in Fig. 3 and discussed above, is simply illustrative and could be structured differently and still remain within the scope of the present invention. For example, processor 301, memory 304, display 308 and interfaces 313 and 314 could be completely or partially contained within the apparatus shown in Fig. 1, rather than in control system 300.

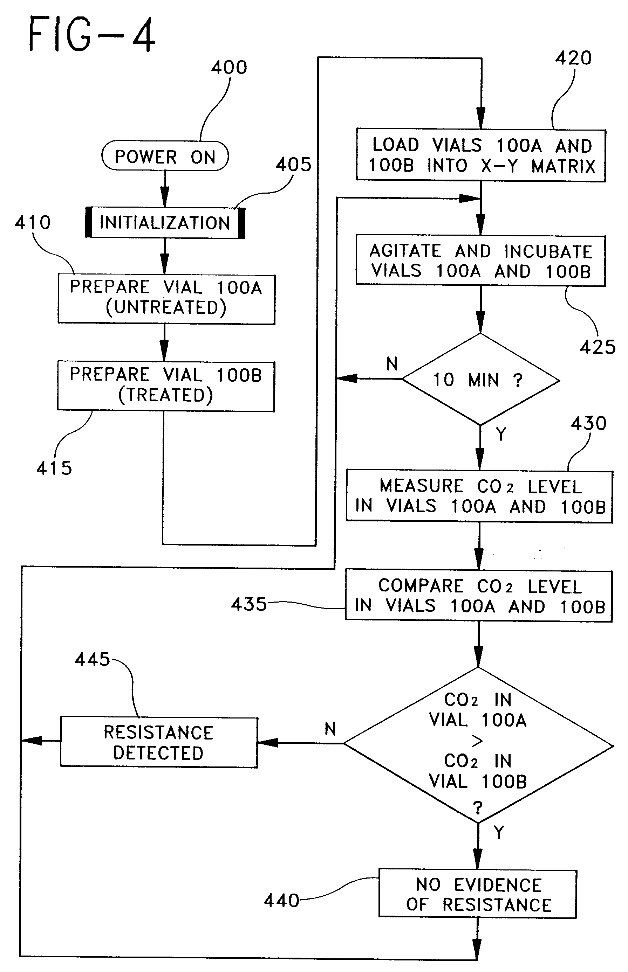

Fig. 4 shows a flow chart of a preferred method of critical specimen resistance testing using the apparatus shown in Figs. 1-3 in accordance with the present invention. When the apparatus is powered-on at step 400, various initialization routines are performed by processor 301 at step 405 to reset the apparatus and ascertain the current status of all the components of the apparatus. At step 410, a first sterile vial 100A containing growth medium 205 and resin medium 207 is injected with a small quantity of blood from a patient through sealing rubber septum 204 at the open upper end of vial 100A. Resin medium 207 absorbs any antibiotics or drugs that may be present in the blood specimen to insure that antimicrobics present in patient's blood do not impair bacterial growth.

At step 415, a second sterile vial 100B containing only growth medium 205 is injected with a small quantity of the patient's blood and an antibiotic prescribed by a physician to treat the patient that provided the blood sample. Then, at step 420, both vials are placed in X-Y matrix 101 and doors 102 of the apparatus are closed.

The processor 301 then performs critical specimen resistance testing on vials 100A and 100B, at step 425, during which the vials are agitated, incubated at a temperature conducive to metabolism of microorganisms, e.g., 37°C, and periodically monitored for bacterial growth. The bacterial growth in each vial 100A and 100B is monitored by respective test assemblies 310 that direct light into the bottom of each vial onto fluorescent-based CO₂ sensors 206 and detect the emitted light using detectors 305. As indicated at step 430, each detector 305 is sampled every ten minutes and the data received by processor 301 from detector interface 314 is stored in memory 304.

The data received by processor 301 from each detector 305 indicates how much CO₂ is present within each vial, which corresponds directly to the growth of bacteria in the vial. At step 435, processor 301 compares the data from detectors 305 associated with vials 100A and 100B, and based upon that comparison determines whether the prescribed therapy is inhibiting microorganism growth. For example, if bacterial growth in vial 100A, which does not contain the antibiotic, is greater than bacterial growth in vial 100B, which contains the antibiotic, there is no evidence of microorganism resistance to the prescribed antibiotic (step 440). However, if bacterial growth in both vials 100A and 100B are similar or growth in treated vial 100B is greater than in untreated vial 100A, microorganism resistance to the prescribed antibiotic may be inferred (step 445). The results of the comparison are then displayed on display 308 or printed at printer 309. The prescribing physician can then be informed of the potential resistance and increase priority given to full susceptibility testing.

Since the above-described apparatus and method of critical specimen resistance testing is capable of collecting data concerning bacterial growth in each vial within a short time, as short as ten minutes, it is possible that the resistance of a microorganism to the prescribed antimicrobic could be detected within a relatively short time period. In any case, as soon as statistically similar growth is detected in treated vial 100B and untreated vial 100A the apparatus is able to detect possible resistance or lack thereof to the prescribed antimicrobic. Therefore, in view of the above, the present invention provides critical data influencing therapy far sooner than tests that are currently available, and probably as early as the "afternoon rounds" of the first day of the patient's hospital stay. As a result, the above method could accelerate successful patient outcome and reduce the chance of a protracted illness because of the resistance of bacteria to a prescribed drug.

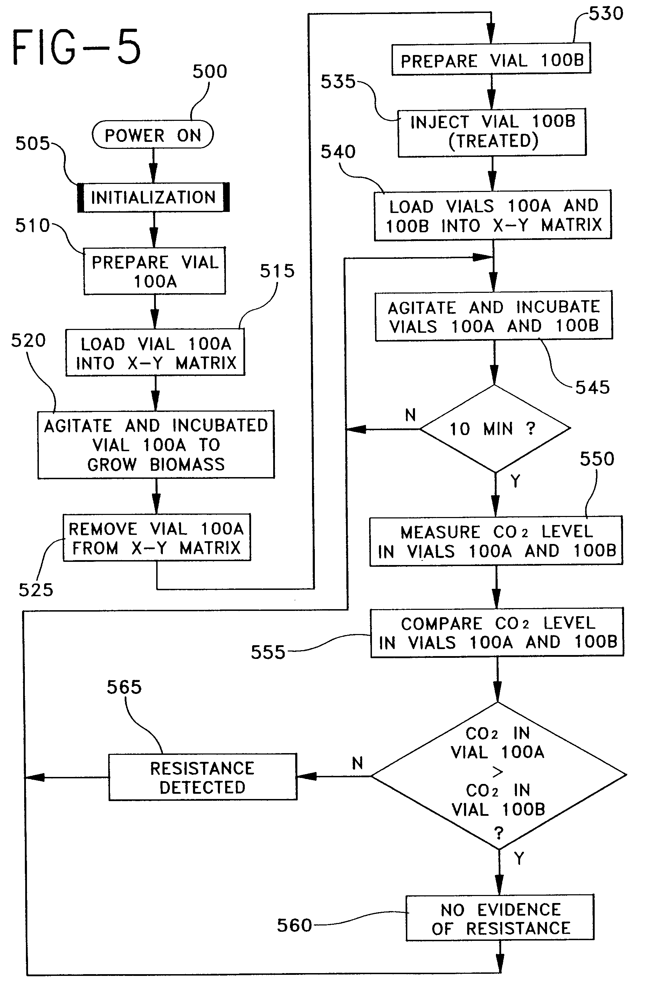

Fig. 5 shows a flow chart of an alternative method in accordance with the present invention. After the apparatus has been powered-on and initialized at steps 500 and 505, a first sterile vial 100A containing growth medium and resin medium is injected with a quantity of blood at step 510, placed into X-Y matrix 101 at step 515, and then agitated, incubated at a temperature conducive to metabolism of microorganisms, e.g., 37°C, and periodically monitored for bacterial growth, at step 520. After a few hours a biomass of the microorganism will have grown in vial 100A. At that time, vial 100A is removed from X-Y matrix 101 at step 525. The culture in vial 100A is then split between the first vial 100A and a second sterile vial 100B at step 530, and the second sterile vial 100B is injected, at step 535, with the antimicrobic prescribed by the physician to treat the patient that provided the blood sample.

At step 540, vials 100A and 100B are loaded into X-Y matrix 101 and the processor 301 then performs critical specimen resistance testing on vials 100A and 100B, at step 545, during which the vials are agitated, incubated at a temperature conducive to metabolism of microorganisms, e.g., 37°C, and periodically monitored for bacterial growth. Then, at steps 550-565, based upon the comparison between the growth rates of microorganisms in each vial, processor 301 determines whether the infecting microorganism is resistant to the prescribed antimicrobic, as described above for steps 430-445, respectively.

In addition, it is to be understood that another sterile vial 100 could be used to contain the untreated specimen in steps 540-565, rather than continuing to use original vial 100A for the untreated specimen.

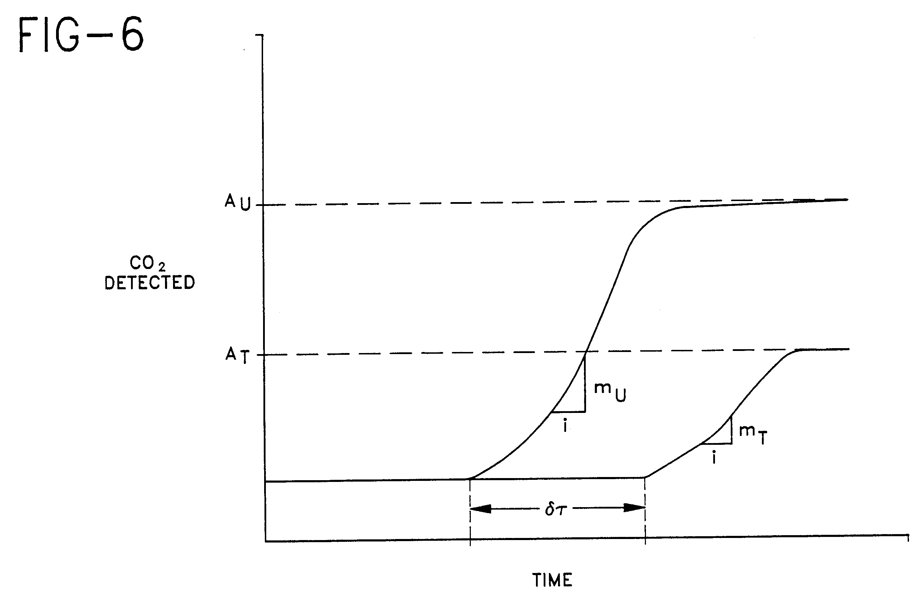

The above-described methods can also be enhanced to identify the genus and species of bacteria. This is performed by first continuously monitoring the growth of a particular bacteria in a treated vial 100B and an untreated vial 100A, as described above, and generating growth response curves, as shown in Fig. 6, for each of a variety of known bacteria in combination with a variety of known antibiotics. Each of these growth response curves is then used to create a database in memory 304 that is then referred to during actual critical specimen resistance testing. Therefore, when performing critical specimen resistance testing, as described above, the growth response curves generated during actual testing are compared with the growth response curves in the database stored in memory 304 to find matching curves and thereby identify the genus and species of the bacteria. The system can also suggest an alternative antibiotic, if needed, using the same database by searching for the antibiotic with the best growth response curve for the particular genus and species of bacteria identified.

Fig. 6 shows a plot that details growth response curves for an untreated vial 100A and a treated vial 100B. The growth response curve for untreated vial 100A is shown at the top of the plot and the growth response curve for treated vial 100B is shown at the bottom of the plot, with each curve relating the amount of CO₂ detected to time. The plot also identifies exemplary growth response curve characteristics that could be used to compare treated vial and untreated vial growth response curves, as discussed above. For example, the maximum amount A_{U} and A_{T} of CO₂ detected, the slope m_{U} and m_{T} of each curve, or the delay δτ in log phase growth between the curves are some curve characteristics that could be used. It is to be understood, however, that the plot in Fig. 6 and the identified characteristics are only for purposes of illustration and not for purposes of limitation and that other suitable curve characteristics could be used to compare the growth response curves for untreated and treated vials.

In addition, once a growth response curve for an untreated vial containing an identified bacteria has been created and stored in memory 304, resistance testing can be performed using only one treated vial. For example, one such method of performing the test using a single vial includes the steps of agitating, incubating, and periodically monitoring the growth of bacteria in the vial. When a logarithmic growth of bacteria is detected, the prescribed antibiotic is injected into the vial and the steps of agitating, incubating and periodically monitoring the growth continue. The resistance of the bacteria to the prescribed antibiotic can then be detected by comparing the periodically measured growth characteristics of the treated vial to those of the stored growth response curve of the untreated vial.

The present invention also provides confirmation for currently available susceptibility tests that take at least 48 hours to generate results. The above-described methods of critical specimen resistance testing based on the present invention would enhance the results of any other susceptibility tests and avoid the problems caused by inaccurate results from those tests.

Accordingly, it will be appreciated that the present invention rapidly provides much needed information concerning the resistance of bacteria to a prescribed antibiotic, and thereby quickly provides information to alter inappropriate therapy, accelerates successful patient outcome, and reduces the chance of a protracted illness because of the resistance of bacteria to a prescribed antibiotic.

In the forgoing discussion, it is to be understood that the above-described embodiment and method of operation are simply illustrative of an apparatus and a method for performing critical specimen resistance testing in accordance with the present invention. Other suitable variations and modifications could be made to the apparatus or methods described and still remain within the scope of the present invention.

## Claims

1. A method of testing the resistance of a microorganism, suspected to be in a fluid specimen, to an antimicrobic comprising the steps of:
introducing a sample of the fluid specimen into a first sterile vial containing a growth medium for encouraging the growth of the microorganism;
introducing another sample of the fluid specimen into a second sterile vial containing the growth medium;
introducing the antimicrobic to inhibit the growth of the suspected microorganism into the second vial;
agitating and incubating the first and second vials at conditions conducive to metabolism of microorganisms;
measuring the concentration of CO₂ in the first vial;
measuring the concentration of CO₂ in the second vial;
comparing the concentration of CO₂ in the first vial to the concentration of CO₂ in the second vial; and
determining whether the antimicrobic is not inhibiting the growth of the suspected microorganism in response to the results of the comparison of CO₂ concentrations.

2. The method of Claim 1, wherein the agitation and incubation of the first and second vials is performed for a period of at least ten minutes.

3. The method of Claim 1, wherein the measurement of CO₂ concentration in the first and second vials is performed periodically.

4. The method of Claim 3, further comprising the steps of:
storing each measurement of the concentration of CO₂ in the first vial to create an untreated vial growth response curve; and
storing each measurement of the concentration of CO₂ in the second vial to create a treated vial growth response curve,
wherein said step of comparing comprises comparing the untreated vial growth response curve and the treated growth response curve.

5. The method of Claim 1, wherein the measurement of CO₂ concentration in the first and second vials is performed at least every ten minutes.

6. The method of Claim 1, further comprising a step of displaying the results of said determination step.

7. The method of Claim 1, wherein the first vial further contains a resin medium for absorbing antimicrobics from the fluid specimen.

8. The method of Claim 1, wherein said step of determining whether the antimicrobic is not inhibiting the growth of the microorganism includes analyzing the results of said comparison step for concluding that the antimicrobic:
a) is inhibiting microorganism growth, if the CO₂ concentration in the first vial is greater than the CO₂ concentration in the second vial; and
b) is not inhibiting microorganism growth, if the CO₂ concentration in the first vial is less than or equal to the CO₂ concentration in the second vial.

9. A method of testing the resistance of a microorganism, suspected to be in a fluid specimen, to an antimicrobic comprising the steps of:
introducing a sample of the fluid specimen into a first sterile vial containing a growth medium for encouraging the growth of the microorganism;
agitating and incubating the first vial at conditions conducive to metabolism of microorganisms until a biomass of the suspected microorganism has been grown in the first vial;
introducing a portion of the biomass from the first vial into a second sterile vial containing the growth medium;
introducing another portion of the biomass from the first vial into a third sterile vial containing the growth medium;
introducing the antimicrobic to inhibit the growth of the suspected microorganism into the third vial;
agitating and incubating the second and third vials at conditions conducive to metabolism of microorganisms;
measuring the concentration of CO₂ in the second vial;
measuring the concentration of CO₂ in the third vial;
comparing the concentration of CO₂ in the second vial to the concentration of CO₂ in the third vial; and
determining whether the antimicrobic is not inhibiting the growth of the suspected microorganism in response to the results of the comparison of CO₂ concentrations.

10. The method of Claim 9, wherein said step of determining whether the antimicrobic is not inhibiting the growth of the suspected microorganism includes analyzing the results of said comparison step for concluding that the antimicrobic:
a) is inhibiting microorganism growth, if the CO₂ concentration in the second vial is greater than the CO₂ concentration in the third vial; and
b) is not inhibiting microorganism growth, if the CO₂ concentration in the second vial is less than or equal to the CO₂ concentration in the third vial.
